(19) Europäisches Patentamt / European Patent Office / Office européen des brevets

(11) **EP 2 194 840 B1**

(12) **EUROPÄISCHE PATENTSCHRIFT**

(45) Veröffentlichungstag und Bekanntmachung des Hinweises auf die Patenterteilung:
**18.05.2011 Patentblatt 2011/20**

(51) Int Cl.:
*A61B 3/12* *(2006.01)*    *G01B 11/25* *(2006.01)*
*G02B 21/00* *(2006.01)*    *G02B 21/06* *(2006.01)*

(21) Anmeldenummer: 08836730.5

(86) Internationale Anmeldenummer:
**PCT/EP2008/007702**

(22) Anmeldetag: **16.09.2008**

(87) Internationale Veröffentlichungsnummer:
**WO 2009/043475 (09.04.2009 Gazette 2009/15)**

(54) **VORRICHTUNG UND VERFAHREN ZUR UNTERSUCHUNG DES AUGENHINTERGRUNDES, INSBESONDERE DER PHOTOREZEPTOREN**

DEVICE AND METHOD FOR EXAMINING THE EYE FUNDUS, ESPECIALLY THE PHOTORECEPTORS

DISPOSITIF ET PROCEDE D'ANALYSE DU FOND DE L'OEIL, EN PARTICULIER DES PHOTORECEPTEURS

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **28.09.2007 DE 102007047460**

(43) Veröffentlichungstag der Anmeldung:
**16.06.2010 Patentblatt 2010/24**

(73) Patentinhaber: **Carl Zeiss Meditec AG 07745 Jena (DE)**

(72) Erfinder: **KUNATH-FANDREI, Gerald 07743 Jena (DE)**

(74) Vertreter: **Beck, Bernard Carl Zeiss AG c/o Patentabteilung Jena Carl-Zeiss-Promenade 10 07745 Jena (DE)**

(56) Entgegenhaltungen:
**EP-A- 1 248 132        EP-A- 1 992 276**
**EP-A1- 0 554 643      WO-A-00/43819**
**WO-A-98/45745        WO-A-2005/044098**
**WO-A-2007/043314    CA-A1- 2 284 299**

- **TAKEAKI YOSHIMURA ET AL: "Topographic Analysis of Ocular Fundus Using the Fourier Transform Method for Projected Grating Images" OPTICAL REVIEW, SPRINGER, BERLIN, DE, Bd. 2, Nr. 5, 1. September 1995 (1995-09-01), Seiten 388-393, XP019353428 ISSN: 1349-9432**
- **NEIL M A A, JUSKAITIS R, AND WILSON T: "Method of obtaining optical sectioning by using structured light in a conventional microscope" OPTICS LETTERS, Bd. 22, Nr. 24, 15. Dezember 1997 (1997-12-15), Seiten 1905-1907, XP000733996**

## Beschreibung

Die vorliegende Erfindung betrifft eine Lösung mit der es möglich ist, neben den üblichen Untersuchungen des Augenhintergrundes auch die Photorezeptoren am menschlichen Auge abzubilden und/oder zu untersuchen, um Erkrankungen frühzeitig diagnostizieren und behandeln zu können. Veränderungen dieser, als Photorezeptoren bezeichneten lichtempfindlichen Sinneszellen eines Auges sind meist erste Anzeichen für Erkrankungen. Ein frühzeitiges Erkennen erhöht dabei die Aussichten für eine erfolgreiche Behandlung um ein Vielfaches.

Das menschliche Auge weist beispielsweise zwei Typen von Photorezeptoren auf: Stäbchen und Zapfen. Während die lichtempfindlicheren Stäbchen das Hell-Dunkel-Sehen ermöglichen, dienen die Zapfen dem Sehen bei Tageslicht und der Farberkennung (rot, grün, blau).

Die Untersuchung und Beurteilung von krankhaften Veränderungen der einsehbaren Teile des Auges, insbesondere der Netzhaut (Retina) und die sie versorgenden Blutgefäße werden als Ophthalmoskopie, Augenspiegelung oder auch Funduskopie bezeichnet. Die der Netzhaut versorgenden Blutgefäße heben sich dabei deutlich von der Retina ab, wobei sich auch die aus der Sehnervpapille (blinder Fleck) entspringenden, hell-rot erscheinenden Arterien von den dunkel-rot erscheinenden Venen abheben.

In der Ophthalmoskopie sind zwei verschiedene Verfahrensweisen üblich. Während die so genannte direkten Ophthalmoskopie, bei der das Ophthalmoskop in einen Abstand von etwa 10cm, d. h. unmittelbar vor das Patientenauge gebracht und bei einer bis zu 15-fachen Vergrößerung ein Bildwinkel von 10 bis 15° erreicht wird, eher zur Untersuchung von Details, wie Sehnervenkopf, Gefäßursprünge und der gelbe Fleck (Makula lutea) geeignet ist, dient die indirekten Ophthalmoskopie, bei einer Entfernung von ca. 50 cm, einer 2- bis 5- fachen Vergrößerung und einem Bildwinkel von 25 bis 40° der Betrachtung und Untersuchung des Augenhintergrundes im Überblick. Von den meisten Augenärzten wird die indirekte Ophthalmoskopie bevorzugt, weil aufgrund der geringeren Vergrößerung ein wesentlich besserer Überblick und im Gegensatz zur direkten Ophthalmoskopie eine stereoskopische (3D) Beurteilung möglich ist. Außerdem kann die indirekte Ophthalmoskopie auch an der Spaltlampe, die bei Augenärzten als Standardgerät für Untersuchungen am Auge gilt, durchgeführt werden. Mit einer Spaltlampe kann das Netzhautbild vergrößert bzw. unter Projektion eines Lichtspaltes, mit noch stärkerem 3D-Effekt beurteilt werden. Allerdings ist die Untersuchung mit einer Spaltlampe nicht dazu geeignet, Details der Retina zu beobachten, da die Auflösung der Optik einer Spaltlampe dazu nicht ausreichend ist.

Neben der Ophthalmoskopie haben sich zur detaillierten, optischen Untersuchung des Augenhintergrundes OCT-Anordnungen (Abkürzung für Optical Coherence Tomography) etabliert. OCT ist ein Untersuchungsverfahren, bei dem zeitlich kurzkohärentes Licht mit Hilfe eines Interferometers zur Entfernungsmessung der im Auge vorhandenen reflektiven Materialien eingesetzt wird.

Das Grundprinzip der OCT basiert auf der Weißlichtinterferometrie, bei der die Laufzeit eines Signals mit Hilfe eines Interferometers (meist Michelson-Interferometer) miteinander verglichen wird. Dabei wird ein Arm des interferometers mit bekannter optischer Weglänge (= Referenzarm) als Referenz zum Messarm herangezogen.

Die Interferenz der Signale (optische Kreuzkorrelation) aus beiden Armen ergibt ein Interferenzmuster, aus dem die relative optische Weglänge innerhalb eines A-Scans (einzelnes Tiefensignal) herausgelesen werden kann. In den eindimensionalen Rasterverfahren wird der Strahl dann transversal in einer oder zwei Richtungen geführt, womit sich ein flächiger B-Scan oder ein dreidimensionales Tomogramm (C-Scan) aufnehmen lässt.

Die herausragende Eigenschaft der OCT ist die Entkoppelung der transversalen von der longitudinalen Auflösung. In der konventionellen Lichtmikroskopie hängt sowohl die axiale Auflösung (in der Tiefe) als auch die transversale (seitliche) Auflösung von der Fokussierung des Lichtstrahles ab. Der Parameter für die Fokussierbarkeit ist die numerische Apertur. Bei der OCT ist die Auflösung nur durch die Bandbreite des verwendeten Lichtes begrenzt. Somit kann eine hohe Auflösung, mit der kleine Details aufgelöst werden können, mit einer großen Bandbreite (weitem Spektrum) erreicht werden.

Die Anwendungsbereiche von OCT liegen primär in der Medizin und dort vor allem in der Augenheilkunde, sowie zur frühzeitigen Krebsdiagnose und zur Hautuntersuchung. Hier werden Reflexionen an Grenzflächen von Materialien mit unterschiedlichen Brechzahlen ausgemessen und so ein dreidimensionales Bild rekonstruiert. Eine solche Rekonstruktion wird als Tomografie bezeichnet.

Der Haupteinsatz liegt derzeit bei der Untersuchung des Augenhintergrundes, bzw. des hinteren Augenabschnittes, da konkurrierende Technologien wie z. B. das Konfokalmikroskop die feine Schichtstruktur der ca. 250-300 $\mu$m dicken Netzhaut aufgrund der geringen Pupillengröße und des großen Abstandes von Hornhaut zur Netzhaut nur unzureichend abbilden können. Der wesentliche Vorteil der OCT liegt nicht zuletzt in der berührungslosen Messung, da damit Infektionsrisiken und die psychische Belastung weitgehend vermieden werden.

Als weiteres Verfahren zur detaillierten, optischen Untersuchung des Augenhintergrundes hat sich auch die Fundusautofluoreszenz (FAF) etabliert. Bei diesem Verfahren können in vivo Lipofuszinakkumulationen im lysosomalen Kompartiment des einschichtigen retinalen Pigmentepithels (RPE) nichtinvasiv erfasst werden.

Von A. Bindewald u. a. wird in [1] eine weiter entwickelte Methode zur Scanning-Laser-Ophthalmoskopie beschrieben. Hierbei werden bei Verwendung konfokaler Scanning-Laser-Ophthalmoskope (cSLO) auf der Basis von Festkörperlasern zur Generierung des Exzitationslaserlichts (488 nm) Auflösung von bis zu 5 μm/Pixel erreicht, so dass sich Veränderungen der topographischen FAF-Intensitätsverteilung bei verschiedenen retinalen Pathologien inklusive altersabhängiger Makuladegeneration, Makulaödem und genetisch determinierten Retinopathien zeigen. Mittels interner Fixationskontrolle, Vergrößerung des Fokusbereichs, verbesserter Optik und neuer Laserquelle ergeben sich Vorteile in der klinischen Anwendung. Eine verbesserte Bildqualität für FAF-Aufnahmen mit dem neuen cSLO ist sowohl für die klinische Diagnostik und die präzise Phänotypisierung von Netzhauterkrankungen für wissenschaftliche Fragestellungen als auch für künftiges Therapiemonitoring auf RPE-Zellebene von Bedeutung.

Mit der methodischen Weiterentwicklung der konfokaler Scanning-Laser-Ophthalmoskopie erfolgt die Gewinnung von FAF-Aufnahmen mit einer bislang nicht erreichten Bildqualität, die sogar die Abgrenzung einzelner RPE-Zellen in vivo erlaubt. Neben sichereren Aussagen bei bisherigen Anwendungen ergeben sich damit auch Möglichkeiten für neue therapeutische Verfahren.

Nachteilig bei diesen beiden Verfahren wirkt sich aus, dass diese auf keinen bei Augenärzten üblichen Standardgeräten basieren und dass die Verfahren extrem teuer sind.

Als übliche Standardgeräte bei Augenärzten gelten sowohl die Spaltlampe als auch die Funduskamera. Während sich die Spaltlampe eher mit der Untersuchung der vorderen Augensegmente befasst, ist die Funduskamera für die Untersuchung des Augenhintergrundes vorgesehen. Etabliert haben sich dabei Funduskameras, die zur Dokumentation über Bildaufzeichnungseinheiten verfügen. Die bildliche Darstellung der Netzhaut des menschlichen Auges ist für die Diagnosestellung ein wichtiges Hilfsmittel. Die technische Realisierung von Aufnahmen des Augenhintergrundes ist jedoch auf Grund der optischen Beschaffenheit des Auges nicht trivial.

Dabei verzichten neue Verfahren auf den Einsatz von Pupillen erweiternden Maßnahmen beim Patienten und arbeiten mit infraroter Beleuchtung. Die Qualität der Befundbilder hängt einerseits wesentlich von der optischen Anordnung der Funduskamera ab, andererseits limitieren die optischen Eigenschaften des Auges selbst, als Teil des Abbildungsstrahlenganges, die erreichbaren Ergebnisse. Moderne Funduskameras verfügen neben einer digitalen Bildaufnahmeeinheit zusätzlich über Bildbearbeitungs- und Archivierungssysteme. Zur Untersuchung des Augenhintergrundes mit fluoreszierenden Lösungen, die dem Blut des Patienten beigegeben werden, sind im Strahlengang entsprechende Anregungs- und Sperrfilter vorhanden, die bei Bedarf in den Strahlengang eingeschwenkt werden.

Da der Betrachtungswinkel klassischer Funduskameras bei etwa 60° liegt, sind detaillierte Untersuchungen der Photorezeptoren am menschlichen Auge nicht möglich, zumal die Auflösung deren Optik dazu nicht ausreichend ist.

Durch spezielle optische Anpassungen ließen sich zwar die üblichen Bildwinkeln beim Fundus Imaging von 45 bis 60° verkleinern und somit kleinere Ausschnitte der menschlichen Retina mit entsprechend höherer Auflösungen abbilden, jedoch lassen sich selbst bei höherer Auflösung die Strukturen der Photorezeptoren nicht sichtbar machen, weil die Tiefenschärfe der Funduskameras zu gering ist. Um die Photorezeptoren der menschlichen Retina abbilden zu können, sind transversale Auflösungen von kleiner 5 μm notwendig. Die beim Fundus Imaging mittels Funduskameras zusätzlich mit abgebildeten out-offocus Schichten der Retina wirken sich zusätzlich nachteilig auf die Abbildungsqualität aus.

Von Gustafsson wird in [2] und WO 1996/24082 A1 beschrieben, dass in einem Fluoreszenzmikroskop die laterale Auflösung um den Faktor zwei vergrößert werden kann, indem die Probe mit einer räumlich strukturierten Beleuchtungsquelle beleuchtet wird. Durch die Beleuchtung mit einer Reihe von Erregungslichtmustern werden normalerweise unzugängliche, hochauflösende Informationen in das beobachtete Bild kodiert. Die gespeicherten Bilder werden linear verarbeitet, um die neuen Informationen zu extrahieren und eine Rekonstruktion mit doppelter Auflösung zu produzieren. Anders als in der konfokalen Mikroskopie wird dabei das gesamte Emissionlicht verwendet, so dass im Vergleich zur konfokalen Mikroskopie Bilder mit erhöhter Klarheit erzeugt werden.

Nach dem Stand der Technik sind weiterhin Lösungen bekannt, bei denen zu untersuchende Proben mit periodischen Mustern beleuchtet werden, um eine Auflösungssteigerung zu erreichen.

Die in der US 5,867,604 A beschriebene Lösung betrifft ein System zur Verbesserung der Auflösung von Abbildungssystemen mittels Verwendung einer Beleuchtung mit periodischem Muster. Dabei wird insbesondere die Beleuchtungsphase verändert, um aus den empfangenen Streulichtbildem die Amplitude und die Phasendaten zu extrahieren und synthetische, dreidimensionale Bilder zu erzeugen. Die periodischen Beleuchtungsmuster können sowohl von Beugungsgittern oder auch auf interferometrischem Wege erzeugt werden. In einer besonders vorteilhaften Ausgestaltung wird die Anwendung des beschriebenen Verfahrens in einem konfokalen Mikroskop beschrieben.

Auch in der WO 1998/045745 A1 wird eine Lösung beschrieben, die ein System zur Verbesserung der Auflösung von Abbildungssystemen mittels Verwendung einer Beleuchtung mit periodischem Muster betrifft. Hierbei wird das Beleuchtungsmuster kontinuierlich oder diskret bewegt, so dass mindestens drei Bilder der Probe mit unterschiedlicher Phasenlage des Beleuchtungsmusters aufgenommen werden können. Von einer Auswerteeinheit werden die räumlichen Muster aus den Bildern entfernt, wodurch ein optisch in Schnitte unterteiltes Bild der Probe erzeugt wird. In einer besonders vorteilhaften Ausgestaltung wird die Anwendung des beschriebenen Verfahrens in einem herkömmlichen Mikroskop beschrieben.

Beide Beschreibungen enthalten zwar Hinweise darauf, dass die Lösungen auch für andere optische Abbildungssysteme verwendet werden können; eine spezielle Anwendung wie beispielsweise in der Ophthalmologie ist jedoch nicht erwähnt.

In der US 5,116,115 A wird ein Verfahren und eine Anordnung zum Messen der Topographie der Kornea eines Auges beschrieben. Hierbei wird ein dünnes, flexibles, reflektierendes Material auf die zu untersuchende Hornhaut gesetzt, so dass sich dieses genau an die Form der Hornhaut anpasst. Dadurch ist es möglich die Form der Hornhaut durch das projizierte Muster zu erfassen. Dazu werden strukturierte, sinusförmige Muster mit unterschiedlichen Phasenlagen auf die Kornea projiziert und von einem Detektor aufgenommen. Aus den digitalisierten Bildern bei unterschiedlichen Phasenlagen wird von einer Computereinheit ein Höhenprofil berechnet. Anhand des ermittelten Höhenprofils kann dann die Topographie der Hornhaut angezeigt werden. Das Wesen der hier beschriebenen Lösung besteht in der Bestimmung der Topographie der Kornea eines Auges. Ein Hinweis auf andere Anwendungen am Auge ist auch hier weder offenbart noch nahe gelegt.

Verfahren und Anordnungen zur mikroskopischen Abbildung, bei denen Gegenstände von einer Lichtquelle mit periodischen Mustern beleuchtet werden, sind in US 6,376,818 B1 und EP 1 412 804 B1 beschrieben. Hierbei besteht das periodische Muster aus einem Streifenmuster. Mittels eines Mikroskops werden mindestens drei Bilder des Gegenstandes bei unterschiedlicher, räumlicher Phasenlage des Musters aufgenommen und an eine Auswerteeinheit übermittelt. In Auswertung dieser drei Bilder wird ein dreidimensionales Bild der Volumenstrukturen des Gegenstandes abgeleitet, welches im Allgemeinen nur Infokus-Details enthält. Mit der vorgeschlagenen Lösung wird eine Anordnung und ein Verfahren zur Erzeugung von dreidimensionalen Bildern eines zu untersuchenden Gegenstandes bereit gestellt, die in einer ähnlichen Weise zu den konfokalen Bildern, im wesentlichen nur Infokus-Detail enthalten. Die Beschreibung enthält zwar einen Hinweis darauf, dass die Lösung insbesondere auch in biomedizinischen Anwendungen benutzt werden kann; eine spezielle Anwendung wie beispielsweise in der Ophthalmologie ist jedoch nicht näher ausgeführt.

In [3] wird von Douglas Starkley ein optisches Projektionssystem zur Detektion von Glaukomerkrankungen beschrieben. Bei dieser Lösung werden veränderbare, von einem Interferometer erzeugte Muster auf die Netzhaut des zu untersuchenden Auges projiziert. Die beim Auftreffen des Lichtes auf die Netzhaut entstehenden elektrischen Potenziale werden mit Hilfe von Elektroden als Elektroretinogramm (ERG) festgehalten. Das ERG zeigt die Summe der Antworten der gesamten Netzhaut. Durch die Projektion bestimmter Muster auf die Netzhaut können insbesondere die inneren Netzhautschichten beurteilt werden. Dazu wurde von Starkley eine Vorrichtung entwickelt, bei der von einem Laser-Interferometer sinusförmige Muster erzeugt und auf die Netzhaut projiziert werden. Das dabei aufgezeichnete Elektroretinogramm wird auch als PERG (Pattem-Electroretinogram) bezeichnet. Während bei früher verwendeten Lösungen die Muster entweder über Projektoren oder Fernsehschirme dargestellt wurden, erzeugt die Muster bei Starkley, einschließlich der Änderungen hinsichtlich Kontrasts, Intensität und räumliche/zeitliche Frequenz ein Laser-Interferometer. Dadurch konnten sowohl Verzerrung als auch chromatische Abweichungen wesentlich reduziert werden. Die bei Starkley erzeugten veränderbaren Muster dienen ausschließlich der Erzeugung von Elektroretinogrammen, insbesondere PERG. Ein Hinweis auf andere Anwendungen am Auge ist weder offenbart noch nahe gelegt.

Literatur:

[1] Bindewald, A. u. a.; "cSLO-Fundusautofluoreszenz-Imaging. Methodische Weiterentwicklung der konfokalen Scanning-Laser-Ophthalmoskopie ", Der Ophthalmologe 3/2005, Pt. 102, pp. 259-264

[2] Gustafsson, M. G. L.; "Surpassing the lateral resolution limit by a factor of two using structured illumination microscopy", Journal of Microscopy, Vol. 198, Pt. 2, 2000, pp. 82-87

[3] Starkey, Douglas E.; Taboada, John; Peters, Daniel; "Image projection optical system for measuring pattern electroretinograms", Proc. SPIE Vol. 2126, 06/1994, p. 271-282,

Der vorliegenden Erfindung liegt die Aufgabe zugrunde eine Lösung zu entwickeln, die die Nachteile des Standes der Technik behebt und mit der es möglich ist, neben den üblichen Untersuchungen des Augenhintergrundes auch die Photorezeptoren am menschlichen Auge abzubilden und/oder zu untersuchen, wobei die Lösung auf einem bei Augenärzten üblichen Standardgerät basieren soll.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Mit der vorgeschlagenen, auf einer Funduskamera gemäß des Präambel von Anspruch 1 basierenden, technischen Lösung sind Untersuchung des Augenhintergrundes sowie der Photorezeptoren am menschlichen Auge möglich. Obwohl die hier beschriebene Lösung auf einer Funduskamera basiert, ist die Lösung prinzipiell auch für andere ophthalmologische Geräte verwendbar.

**EP 2 194 840 B1**

Die Erfindung wird nachfolgend anhand eines Ausführungsbeispieles näher beschrieben. Dazu zeigt:

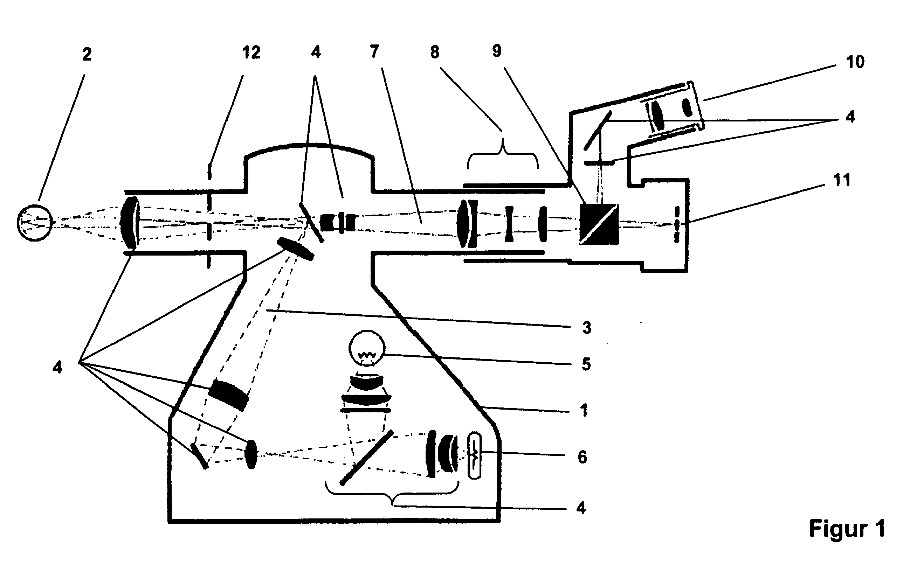

**Figur 1:** den prinzipiellen Aufbau einer erfindungsgemäßen Funduskamera.

Die erfindungsgemäße Vorrichtung zur Untersuchung des Augenhintergrundes sowie der Photorezeptoren am menschlichen Auge besteht aus einem Beleuchtungsstrahlengang, in dem neben optischen Bauelementen zur Strahlformung und/oder -führung mindestens eine Beleuchtungseinheit zur Realisierung einer stetigen und einer Blitzbeleuchtung vorhanden ist, und einem Beobachtungs- und Abbildungsstrahlengang, der neben optischen Bauelementen zur Strahlformung und/oder -führung über eine Vorrichtung zur Veränderung der Vergrößerung und über einen Strahlteiler zur Aufteilung des Strahlenganges in Richtung der Okulare und in Richtung des Bildaufnahmesensors verfügt. Hierbei ist in einer zur Objektebene konjugierten Ebene, im Beobachtungs- und Abbildungsstrahlengang ein in unterschiedliche Lagen drehbares, optisches Gitter vorhanden, deren Bewegung mit der als Blitzbeleuchtung fungierenden Beleuchtungseinheit synchronisiert ist, so dass vom Bildaufnahmesensor eine schnelle Abfolge von Abbildern des Augenhintergrundes bei unterschiedlicher Stellung des Gitters aufgenommen und an eine vorhandene Auswerteeinheit weitergeleitet werden.

Das im Beobachtungs- und Abbildungsstrahlengang vorhandene, optische Gitter weist vorzugsweise eine periodische Struktur auf.

Der Bildaufnahmesensor ist so ausgebildet, dass er eine schnelle Abfolge von 3 Abbildern des Augenhintergrundes bei jeweils um 120° verdrehter Stellung des Gitters aufnehmen und an die Auswerteeinheit weiterleiten kann. Vorzugsweise kann der Bildaufnahmesensor die schnelle Abfolge von Abbildern des Augenhintergrundes bei unterschiedlicher Stellung des Gitters innerhalb in einer Zeit von weniger als 300ms aufnehmen und an die Auswerteeinheit weiterleiten.

Hierzu zeigt **Figur 1** den prinzipiellen Aufbau einer erfindungsgemäßen Funduskamera. Die erfindungsgemäße Funduskamera **1** zur Untersuchung des Augenhintergrundes sowie der Photorezeptoren am menschlichen Auge **2** besteht aus einem Beleuchtungsstrahlengang **3**, in dem neben optischen Bauelementen zur Strahlformung und/oder -führung **4** eine Beleuchtungseinheit **5** zur stetigen Beleuchtung und eine Beleuchtungseinheit **6** zur Blitzbeleuchtung vorhanden sind, und einem Beobachtungs- und Abbildungsstrahlengang **7**, der neben optischen Bauelementen zur Strahlformung und/oder -führung **4** über eine Vorrichtung zur Veränderung der Vergrößerung **8** und über einen Strahlteiler **9** zur Aufteilung des Beobachtungs- und Abbildungsstrahlengang **7** in Richtung der Okulare **10** und in Richtung des Bildaufnahmesensors **11** verfügt. Hierbei ist in einer zur Objektebene konjugierten Ebene, im Beobachtungs- und Abbildungsstrahlengang **7** ein in unterschiedliche Lagen drehbares, optisches Gitter **12** vorhanden, dessen Bewegung mit der Beleuchtungseinheit **6** zur Blitzbeleuchtung synchronisiert ist, so dass vom Bildaufnahmesensor **11** eine schnelle Abfolge von Abbildern des Augenhintergrundes des Auges **2** bei unterschiedlicher Stellung des Gitters **12** aufgenommen und an eine (nicht dargestellte) Auswerteeinheit weitergeleitet werden. Hierbei sind sowohl die Beleuchtungseinheit **5** zur stetigen Beleuchtung als auch die Beleuchtungseinheit **6** zur Blitzbeleuchtung als Weißlichtquellen ausgeführt. In einer vorteilhaften Ausgestaltung sind beide Beleuchtungseinheiten **5** und **6** als LED bzw. LED-Array ausgeführt.

Die vom Bildaufnahmesensor **11** in schneller Abfolge aufgenommenen Abbilder des Augenhintergrundes des Auges **2** bei unterschiedlicher Stellung des Gitters **12** werden an die Auswerteeinheit übermittelt, die die Abbilder verarbeitet, um die neuen Informationen zu extrahieren und ein rekonstruiertes Abbild mit höherer Auflösung zu produzieren.

Bei dem erfindungsgemäßen Verfahren zur Untersuchung des Augenhintergrundes sowie der Photorezeptoren am menschlichen Auge wird das zu untersuchende Auge über einen Beleuchtungsstrahlengang, der neben optischen Bauelementen zur Strahlformung und/oder -führung mindestens über eine Beleuchtungseinheit zur Realisierung einer stetigen und einer Blitzbeleuchtung verfügt, beleuchtet und über einen Beobachtungs- und Abbildungsstrahlengang, der neben optischen Bauelementen zur Strahlformung und/oder - führung, über eine Vorrichtung zur Veränderung der Vergrößerung und über einen Strahlteiler zur Aufteilung des Strahlenganges in Richtung vorhandener Okulare und in Richtung eines Bildaufnahmesensors verfügt, beobachtet und/oder abgebildet. Vom Bildaufnahmesensor wird dabei eine schnelle Abfolge von Abbildern des Augenhintergrundes bei unterschiedlicher Stellung eines in einer zur Objektebene konjugierten Ebene, im Beobachtungs- und Abbildungsstrahlengang und in unterschiedliche Lagen drehbaren, optischen Gitters aufgenommen und an eine vorhandene Auswerteeinheit weitergeleitet. Dabei befinden sich das Gitter, das Objekt und die Zwischenbildebene in zueinander konjugierten Ebenen (Schärfeebenen).

Die Bewegung des optischen Gitters, welches vorzugsweise über eine periodische Struktur verfügt, ist hierbei mit der als Blitzbeleuchtung fungierenden Beleuchtungseinheit synchronisiert. Vom Bildaufnahmesensor wird vorzugsweise eine schnelle Abfolge von 3 Abbildern des Augenhintergrundes bei jeweils um 120° verdrehter Stellung des Gitters aufgenommen und an die Auswerteeinheit weitergeleitet. Dabei ist der Bildaufnahmesensor so ausgeführt, dass die schnelle Abfolge von Abbildern des Augenhintergrundes innerhalb einer Zeit von weniger als 300ms aufgenommen und an eine vorhandene Auswerteeinheit weitergeleitet wird.

In einer vorteilhaften Ausgestaltung des Verfahrens werden die beschriebenen Verfahrensschritte mit einer modifizierten Funduskamera durchgeführt, wobei sowohl die stetige als auch die Blitzbeleuchtung mit Weißlicht erfolgt.

Vorzugsweise wird das Weißlicht dabei von LED's bzw. einem LED-Array erzeugt.

Mit Hilfe von drei kurzen Lichtblitzen der Beleuchtungseinheit zur Realisierung der Blitzbeleuchtung und der entsprechend synchronisierten Bewegung des Gitters, werden Abbilder bei unterschiedlichen Phasenlagen des Gitters aufgenommen und von der Auswerteeinheit weiter verarbeitet.

Der durch die Überlagerung der Gitterstruktur mit der Objektstruktur im detektierten Bild sichtbare "Gitterkontrast" ist ein Maß für die "Konfokalität" der Anordnung. Lediglich die Bereiche des Objekts, die sich in der Schärfeebene des Objektivs befinden, werden strukturiert abgebildet. Wird nun durch eine Verschiebung des Gitters der resultierende Kontrast für jeden Bildpunkt berechnet, so erhält man einen optischen Schnitt des Objekts.

Von der Auswerteeinheit wird die Detektion von derart strukturierten Bildern des Objekts sequentiell durchgeführt. Vorzugsweise werden Abbilder des Augenhintergrundes bei 3 verschiedenen, relativen Orts-Phasen des Gitters aufgenommen. Bei einer relativen Phasendifferenz von jeweils 120° zwischen den Einzelbildem kann von der Auswerteeinheit mit einem einfachen Algorithmus der defokussierte Bildanteil entfernt werden, wobei dadurch der Tiefenkontrast des detektierten Objekts erhöht wird.

Der Algorithmus zur Berechnung des synthetischen, optischen Schnittbildes des Augenhintergrundes entspricht der Berechnung der Modulationstiefe des mit der optischen Trägerfrequenz (Gitterfrequenz) kodierten Objekts. Bei einem Rechteckgitter ist zur Minimierung der höheren Harmonischen der folgende 3-Phasen-Algorithmus anzuwenden:

$$I_{sectioned\,x,y} = 2\,\frac{\sqrt{2}}{3}\left\{\sqrt{\left[I_{x,y}(0)-I_{x,y}\left(\frac{2\pi}{3}\right)\right]^2 + \left[I_{x,y}(0)-I_{x,y}\left(\frac{4\pi}{3}\right)\right]^2 + \left[I_{x,y}\left(\frac{2\pi}{3}\right)-I_{x,y}\left(\frac{4\pi}{3}\right)\right]^2}\right\}$$

indem,

$I_{x,y}$ die Intensität im Bildpunkt (x,y) in den unterschiedlichen Abbildern (Phasenlagen des Gitters) darstellt.

Das Ergebnis ist ein mit bekannten konfokalen Techniken (SLO) vergleichbares optisches Schnittbild des Objekts.

Mit der erfindungsgemäßen Lösung werden eine Vorrichtung und ein Verfahren zur Verfügung gestellt, mit dem sowohl Untersuchungen des Augenhintergrundes als auch der Photorezeptoren des menschlichen Auges möglich sind. Vorzugsweise basiert die Lösung auf einer Funduskamera, welche bei den Augenärzten als Standardgerät gilt, so dass die Lösung eine breite Anwendung finden kann. Durch die Verwendung eines drehbaren Gitters, einer LED-Beleuchtung und einer schnellen Bildaufnahmeeinheit kann auf Basis der damit erzeugten strukturierte Beleuchtung selbst mit einer "einfachen" Funduskamera kontrastreiche Abbilder der Photorezeptoren eines menschlichen Auges erzeugt werden. Die kontrastreichen, in-vivo erzeugten Bilder der Photorezeptoren weisen eine hohe Tiefenschärfe auf und sind durchaus mit den mit bekannten konfokalen Techniken (SLO) erzeugten optisches Schnittbild des Objekts vergleichbar.

## Patentansprüche

1. Vorrichtung zur Untersuchung des Augenhintergrundes, insbesondere der Photorezeptoren am menschlichen Auge, bestehend aus einem Beleuchtungsstrahlengang (3), in dem neben optischen Bauelementen zur Strahlformung und/oder -führung (4) mindestens eine Beleuchtungseinheit (5) zur Realisierung einer stetigen und einer Blitzbeleuchtung (6) vorhanden ist, und einem Beobachtungs- und Abbildungsstrahlengang (7), der neben optischen Bauelementen zur Strahlformung und/oder -führung (4) und einer Vorrichtung zur Veränderung der Vergrößerung (8), über einen Strahlteiler (9) zur Aufteilung des Beobachtungs- und Abbildungsstrahlenganges (7) in Richtung von Okularen (10) der Vorrichtung und in Richtung eines Bildaufnahmesensors (11) der Vorrichtung verfügt,
**dadurch gekennzeichnet, dass**
in einer zur Objektebene konjugierten Ebene im Beobachtungs- und Abbildungsstrahlengang (7) ein in unterschiedliche Lagen drehbares, optisches Gitter (12) vorhanden ist, dessen Bewegung mit der als Blitzbeleuchtung (6) fungierenden Beleuchtungseinheit synchronisiert ist, wobei der Bildaufnahmesensor (11) dazu ausgebildet ist, eine schnelle Abfolge von Abbildern des Augenhintergrundes des Auges (2) bei unterschiedlicher Stellung des Gitters (12) aufzunehmen und an eine Auswerteeinheit der Vorrichtung weiterzuleiten.

2. Vorrichtung nach Anspruch 1, bei der das im Beobachtungs- und Abbildungsstrahlengang (7) vorhandene, optische Gitter (12) eine periodische Struktur aufweist.

3. Vorrichtung nach Anspruch 1 oder 2, wobei der Bildaufnahmesensor (11) dazu ausgebildet ist, eine schnelle Abfolge von 3 Abbildern des Augenhintergrundes des Auges (2) bei jeweils um 120° verdrehter Stellung des Gitters (12) aufzunehmen und an die Auswerteeinheit weiterzuleiten.

4. Vorrichtung nach einem der Ansprüche 1 bis 3, wobei der Bildaufnahmesensor (11) dazu ausgebildet ist, die schnelle Abfolge von Abbildern des Augenhintergrundes des Auges (2) bei unterschiedlicher Stellung des Gitters (12) innerhalb einer Zeit von maximal 300ms aufzunehmen und an die Auswerteeinheit weiterzuleiten.

5. Vorrichtung nach mindestens einem der vorgenannten Ansprüche, bei dem die Vorrichtung zur Untersuchung des Augenhintergrundes, insbesondere der Photorezeptoren am menschlichen Auge (2) eine Funduskamera (1) ist.

6. Vorrichtung nach Anspruch 5, bei der die Beleuchtungseinheit (5) so ausgeführt ist, dass sowohl die stetige als auch die Blitzbeleuchtung (6) auf einer Weißlichtquelle basieren.

7. Verfahren zur Untersuchung des Augenhintergrundes, insbesondere der Photorezeptoren am menschlichen Auge, bei dem das zu untersuchende Auge (2) über einen Beleuchtungsstrahlengang (3), der neben optischen Bauelementen zur Strahlformung und/oder -führung (4) mindestens über eine Beleuchtungseinheit zur Realisierung einer stetigen (5) und einer Blitzbeleuchtung (6) verfügt, beleuchtet und über einen Beobachtungs- und Abbildungsstrahlengang (7), der neben optischen Bauelementen zur Strahlformung und/oder -führung (4) und eine Vorrichtung zur Veränderung der Vergrößerung (8), über einen Strahlteiler (9) zur Aufteilung des Beobachtungs- und Abbildungsstrahlenganges (7) in Richtung vorhandener Okulare (10) und in Richtung eines Bildaufnahmesensors (11) verfügt, beobachtet und/oder abgebildet wird,

**dadurch gekennzeichnet, dass**
vom Bildaufnahmesensor (11) eine schnelle Abfolge von Abbildern des Augenhintergrundes des Auges (2) bei unterschiedlicher Stellung eines in einer zur Objektebene konjugierten Ebene des Beobachtungs- und Abbildungsstrahlenganges (7) angeordneten und in unterschiedliche Lagen drehbaren, optischen Gitters (12) aufgenommen und an eine vorhandene Auswerteeinheit weitergeleitet wird, wobei die Bewegung des optischen Gitters (12), welches vorzugsweise über eine periodische Struktur verfügt, mit der als Blitzbeleuchtung (6) fungierenden Beleuchtungseinheit synchronisiert ist.

8. Verfahren nach Anspruch 7, bei dem vom Bildaufnahmesensor (11) eine schnelle Abfolge von 3 Abbildern des Augenhintergrundes des Auges (2) bei jeweils um 120° verdrehter Stellung des Gitters (12) aufgenommen und an die Auswerteeinheit weitergeleitet wird.

9. Verfahren nach mindestens einem der Ansprüche 7 und 8, bei dem vom Bildaufnahmesensor (11) die schnelle Abfolge von Abbildern des Augenhintergrundes des Auges (2) bei unterschiedlicher Stellung des Gitters (12) innerhalb einer Zeit von weniger als 300ms aufgenommen und an die Auswerteeinheit weitergeleitet wird.

10. Verfahren nach Anspruch 8, bei dem von der Auswerteeinheit folgender Algorithmus zur Berechnung des synthetischen, optischen Schnittbildes des Augenhintergrundes des Auges (2) angewendet wird, bei dem $I_{x,y}$ der Intensität im Bildpunkt (x,y) in den unterschiedlichen Abbildern entspricht:

$$I_{sectioned\ x,y} = 2\frac{\sqrt{2}}{3}\left\{\sqrt{\left[I_{x,y}(0)-I_{x,y}\left(\frac{2\pi}{3}\right)\right]^2 + \left[I_{x,y}(0)-I_{x,y}\left(\frac{4\pi}{3}\right)\right]^2 + \left[I_{x,y}\left(\frac{2\pi}{3}\right)-I_{x,y}\left(\frac{4\pi}{3}\right)\right]^2}\right\}$$

11. Verfahren nach mindestens einem der Ansprüche 7 bis 10, bei dem als Vorrichtung zur Untersuchung des Augenhintergrundes, insbesondere der Photorezeptoren am menschlichen Auge eine Funduskamera (1) Verwendung findet.

12. Verfahren nach Anspruch 11, bei dem sowohl die stetige (5) als auch die Blitzbeleuchtung (6) mit Weißlicht erfolgt.

**Claims**

1. Device for examining the eye fundus, especially the photoreceptors in the human eye, consisting of an illumination beam path (3), in which there is at least one illumination unit (5) for implementing a constant and a flash illumination (6) in addition to optical components for beam shaping and/or beam guiding (4), and an observation and imaging beam path (7), which comprises a beam splitter (9) for splitting the observation and imaging beam path (7) in the direction of eyepieces (10) of the device and in the direction of an image-recording sensor (11) of the device in addition to optical components for beam shaping and/or beam guiding (4) and a device for modifying the magnification (8),

   **characterized in that**

   there is an optical grating (12), which can be rotated into different positions, in a plane in the observation and imaging beam path (7), which plane is conjugate to the object plane, the movement of which grating is synchronized with the illumination unit acting as a flash illumination (6), wherein the image-recording sensor (11) is designed to record a quick succession of images of the eye fundus of the eye (2) at different positions of the grating (12) and to transmit said images to an evaluation unit of the device.

2. Device according to Claim 1, in which the optical grating (12) present in the observation and imaging beam path (7) has a periodic structure.

3. Device according to Claim 1 or 2, in which the image-recording sensor (11) is designed to record a quick succession of 3 images of the eye fundus of the eye (2), with the position of the grating (12) being rotated by 120° in each case, and to transmit said images to the evaluation unit.

4. Device according to one of Claims 1 to 3, in which the image-recoding sensor (11) is designed to record the quick succession of images of the eye fundus of the eye (2) at different positions of the grating (12) within a time of at most 300 ms and to transmit said images to the evaluation unit.

5. Device according to at least one of the preceding claims, in which the device for examining the eye fundus, especially the photoreceptors in the human eye (2), is a fundus camera (1).

6. Device according to Claim 5, in which the illumination unit (5) is embodied such that both the constant and the flash illumination (6) are based on a white-light source.

7. Method for examining the eye fundus, especially the photoreceptors in the human eye, in which the eye (2) to be examined is illuminated by an illumination beam path (3), which comprises at least one illumination unit for implementing a constant (5) and a flash illumination (6) in addition to optical components for beam shaping and/or beam guiding (4), and observed and/or imaged by an observation and imaging beam path (7), which comprises a beam splitter (9) for splitting the observation and imaging beam path (7) in the direction of available eyepieces (10) and in the direction of an image-recording sensor (11) in addition to optical components for beam shaping and/or beam guiding (4) and a device for modifying the magnification (8),
   **characterized in that**
   a quick succession of images of the eye fundus of the eye (2) are recorded by the image-recording sensor (11) at different positions of an optical grating (12), which is arranged in a plane of the observation and illumination beam path (7), which plane is conjugate to the object plane, and can be rotated into different positions, and said images are transmitted to an available evaluation unit, in which the movement of the optical grating (12), which preferably comprises a periodic structure, is synchronized with the illumination unit acting as a flash illumination (6).

8. Method according to Claim 7, in which the image-recording sensor (11) records a quick succession of 3 images of the eye fundus of the eye (2), with the position of the grating (12) being rotated by 120° in each case, and transmits said images to the evaluation unit.

9. Method according to at least one of Claims 7 and 8, in which the image-recording sensor (11) records the quick succession of images of the eye fundus of the eye (2) at different positions of the grating (12) within a time of less than 300 ms and transmits said images to the evaluation unit.

10. Method according to Claim 8, in which the following algorithm is applied by the evaluation unit in order to calculate the synthetic, optical cross section of the eye fundus of the eye (2), in which $I_{x,y}$ corresponds to the intensity in the pixel (x, y) in the various images:

$$I_{sectioned\,x,y} = 2\frac{\sqrt{2}}{3}\left\{\sqrt{\left[I_{x,y}(0)-I_{x,y}\left(\frac{2\pi}{3}\right)\right]^2+\left[I_{x,y}(0)-I_{x,y}\left(\frac{4\pi}{3}\right)\right]^2+\left[I_{x,y}\left(\frac{2\pi}{3}\right)-I_{x,y}\left(\frac{4\pi}{3}\right)\right]^2}\right\}$$

**11.** Method according to at least one of Claims 7 to 10, in which a fundus camera (1) is used as device for examining the eye fundus, especially the photoreceptors in the human eye.

**12.** Method according to Claim 11, in which white light is used for both the constant (5) and the flash illumination (6).

**Revendications**

**1.** Dispositif d'analyse du fond de l'oeil, en particulier des photorécepteurs de l'oeil humain, constitué d'un chemin de rayons d'éclairage (3) dans lequel en plus de composants optiques pour la mise en forme et/ou le guidage des rayons (4), on dispose d'au moins une unité d'éclairage (5) pour la réalisation d'un éclairage continu et par éclairs (6), et d'un chemin de rayons d'observation et d'imagerie (7) qui, en plus de composants optiques pour la mise en forme et/ou le guidage des rayons (4) et d'un dispositif pour la modification du grandissement (8), dispose d'un séparateur de faisceaux (9) pour la séparation du chemin de rayons d'observation et d'imagerie (7) en direction d'oculaires (10) du dispositif et en direction d'un capteur d'images (11) du dispositif, **caractérisé en ce que** dans un plan conjugué au plan objet dans le chemin de rayons d'observation et d'imagerie (7), on dispose d'une grille optique (12) pivotant dans différentes positions, dont le mouvement est synchronisé avec l'unité d'éclairage faisant fonction d'éclairage par éclairs (6), dans lequel le capteur d'images (11) est conçu pour enregistrer une succession rapide d'images du fond de l'oeil de l'oeil (2) pour différentes positions de la grille (12) et pour l'acheminer vers une unité de traitement du dispositif.

**2.** Dispositif selon la revendication 1, dans lequel la grille optique (12) existant dans le chemin de rayons d'observation et d'imagerie (7), présente une structure périodique.

**3.** Dispositif selon la revendication 1 ou 2, dans lequel le capteur d'images (11) est conçu pour enregistrer un succession rapide de 3 images du fond de l'oeil de l'oeil (2) pour des positions de la grille (12) tournées respectivement de 120° et pour l'acheminer vers l'unité de traitement.

**4.** Dispositif selon une des revendications 1 à 3, dans lequel le capteur d'images (11) est conçu pour enregistrer la succession rapide d'images du fond de l'oeil de l'oeil (2) pour différentes positions de la grille (12) dans un délai maximum de 300 ms et pour l'acheminer vers l'unité de traitement.

**5.** Dispositif selon une au moins des revendications précédentes, dans lequel le dispositif d'analyse du fond de l'oeil, en particulier des photorécepteurs de l'oeil humain (2) est une caméra du fond (1).

**6.** Dispositif selon la revendication 5, dans lequel l'unité d'éclairage (5) est réalisée de telle sorte que l'éclairage continu mais aussi par éclairs (6) est basé sur une source de lumière blanche.

**7.** Procédé d'analyse du fond de l'oeil, en particulier des photorécepteurs de l'oeil humain, dans lequel l'oeil à examiner (2) est éclairé par l'intermédiaire d'un chemin de rayons d'éclairage (3) qui en plus de composants optiques pour la mise en forme et/ou le guidage des rayons (4), dispose d'au moins une unité d'éclairage pour la réalisation d'un éclairage continu (5) et par éclairs (6), et dans lequel l'oeil à examiner (2) est observé et reproduit par l'intermédiaire d'un chemin de rayons d'observation et d'imagerie (7) qui en plus de composants optiques pour la mise en forme et/ou le guidage des rayons (4) et d'un dispositif pour la modification du grandissement (8), dispose d'un séparateur de faisceaux (9) pour la séparation du chemin de rayons d'observation et d'imagerie (7) en direction des oculaires disponibles (10) et en direction d'un capteur d'images (11) du dispositif, **caractérisé en ce que** le capteur d'images (11) enregistre une succession rapide d'images du fond de l'oeil de l'oeil (2) pour différentes positions d'une grille optique (12) disposée dans un plan conjugué au plan objet du chemin de rayons d'observation et d'imagerie (7) et pivotant dans différentes positions et l'achemine vers une unité de traitement disponible, le mouvement de la grille optique (12) qui dispose de préférence d'une structure périodique, étant synchronisé avec l'unité d'éclairage faisant fonction d'éclairage par éclairs (6).

8. Procédé selon la revendication 7, dans lequel le capteur d'images (11) enregistre une succession rapide de 3 images du fond de l'oeil de l'oeil (2) pour des positions de la grille (12) tournées respectivement de 120° et l'achemine vers l'unité de traitement.

9. Procédé selon une au moins des revendications 7 et 8, dans lequel le capteur d'images (11) enregistre la succession rapide d'images du fond de l'oeil de l'oeil (2) pour différentes positions de la grille (12) dans un délai inférieur à 300 ms et l'achemine vers l'unité de traitement.

10. Procédé selon la revendication 8, dans lequel l'unité de traitement applique l'algorithme suivant pour le calcul de l'image en coupe optique synthétique du fond de l'oeil de l'oeil (2), dans lequel $I_{x,y}$ correspond à l'intensité du point d'image (x, y) dans les différentes images :

$$I_{\text{yr coord.},x,y} = 2\frac{\sqrt{2}}{3}\left\{\sqrt{\left[I_{x,y}(0)-I_{x,y}\left(\frac{2\pi}{3}\right)\right]^2 + \left[I_{x,y}(0)-I_{x,y}\left(\frac{4\pi}{3}\right)\right]^2 + \left[I_{x,y}\left(\frac{2\pi}{3}\right)-I_{x,y}\left(\frac{4\pi}{3}\right)\right]^2}\right\}$$

11. Procédé selon une au moins des revendications 7 à 10, dans lequel on utilise une caméra du fond (1) comme dispositif d'analyse du fond de l'oeil, en particulier des photorécepteurs de l'oeil humain.

12. Procédé selon la revendication 11, dans lequel l'éclairage continu (5) mais aussi par éclairs (6) a lieu en lumière blanche.

Figur 1

EP 2 194 840 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 199624082 A1
- US 5867604 A
- WO 1998045745 A1
- US 5116115 A
- US 6376818 B1
- EP 1412804 B1

**In der Beschreibung aufgeführte Nicht-Patentliteratur**

- **Bindewald, A. u. a.** cSLO-Fundusautofluoreszenz-Imaging. Methodische Weiterentwicklung der konfokalen Scanning-Laser-Ophthalmoskopie. *Der Ophthalmologe,* Marz 2005, 259-264
- **Gustafsson, M. G. L.** Surpassing the lateral resolution limit by a factor of two using structured illumination microscopy. *Journal of Microscopy,* 2000, vol. 198, 82-87
- **Starkey, Douglas E. ; Taboada, John ; Peters, Daniel.** Image projection optical system for measuring pattern electroretinograms. *Proc. SPIE,* Juni 1994, vol. 2126, 271-282